(19) Europäisches Patentamt
European Patent Office
Office européen des brevets

(11) Numéro de publication: **0 058 591**
**B1**

(12) # FASCICULE DE BREVET EUROPEEN

(45) Date de publication du fascicule du brevet: **03.10.84**

(21) Numéro de dépôt: **82400158.0**

(22) Date de dépôt: **28.01.82**

(51) Int. Cl.³: **C 07 C 51/353**, C 07 C 61/37, C 07 F 5/04, C 07 F 9/113, C 07 F 7/18, C 07 C 43/303

(54) **Procédé d'épimérisation d'acides trans chrysanthémiques et nouveaux intermédiaires obtenus.**

(30) Priorité: **13.02.81 FR 8102828**

(43) Date de publication de la demande:
**25.08.82 Bulletin 82/34**

(45) Mention de la délivrance du brevet:
**03.10.84 Bulletin 84/40**

(84) Etats contractants désignés:
**AT BE CH DE GB IT LI LU NL SE**

(56) Documents cités:
**JOURNAL OF ORGANIC CHEMISTRY, vol. 42, no. 12, 10 juin 1977, S.C. WELCH et al.: "A synthesis of (+,-)-trans-chrysanthemic acid" pages 2108-2111**

(73) Titulaire: **ROUSSEL-UCLAF, 35, boulevard des Invalides, F-75007 Paris (FR)**

(72) Inventeur: **Martel, Jacques, 15, rue Douvillez, F-93140-Bondy (FR)**
Inventeur: **Buendia, Jean, 3, Impasse Emilie, F-94170-Le Perreux sur Marne (FR)**
Inventeur: **Nierat, Jeanine, 44, rue Carnot, F-92150-Suresnes (FR)**

(74) Mandataire: **Tonnellier, Marie-José et al, ROUSSEL-UCLAF 111, route de Noisy Boîte Postale no.9, F-93230 Romainville (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 058 591 B1

ACTORUM AG

## Description

On connaissait jusqu'ici des procédés d'épimérisation de l'acide cischrysanthémique (I) :

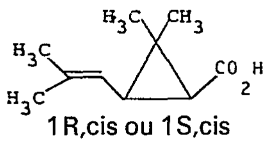

(I)

1 R,cis ou 1 S,cis

ou de ses dérivés, en acide transchrysanthémique.

Il est en effet connu que la forme thermodynamiquement la plus stable est la forme trans, l'équilibre, variable selon les conditions opératoires, pouvant être de l'ordre de 90% de trans pour 10% de cis.

Or les composés de la résine cis ont pris un grand intérêt, notamment après la découverte des propriétés insecticides élevées des esters des acides 1 R,cis-2,2-diméthyl-3-(2,2-dihalovinyl)cyclopropane-1-carboxyliques.

Les chercheurs de la titulaire ont maintenant inventé un procédé d'épimérisation des esters d'alcoyle de l'acide transchrysanthémique en esters d'alcoyle de l'acide cischrysanthémique et plus spécialement d'esters d'acide 1 S,transchrysanthémique en esters d'acide 1 R,cischrysanthémique.

La réalisation d'un tel procédé se heurtait à de graves difficultés, l'équilibre thermodynamique étant en faveur de la forme trans.

Une première étape du procédé selon l'invention est essentiellement caractérisée en ce que l'on fait réagir en milieu anhydre une base forte sur un ester d'alcoyle d'un acide transchrysanthémique (II) :

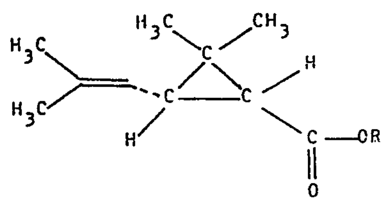

(II)

1 S,trans ou 1 R,trans, dans lequel R représente un radical alcoyle renfermant de 1 à 6 atomes de carbone, pour obtenir un énolate (III) :

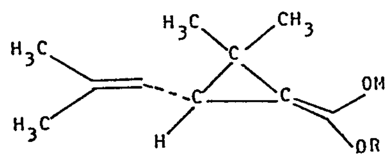

(III)

puis en ce que l'on fait réagir sur l'énolate (III) un réactif Y-A, dans lequel A représente un atome de chlore, de brome ou d'iode ou un autre anion dérivé d'un hydracide et Y représente un radical organique tel que le réactif Y-A soit susceptible de former un cétène de cétal (IV) :

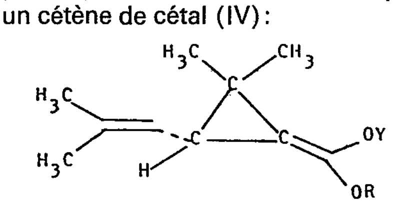

(IV)

La formation du cétène de cétal (IV) à partir de l'énolate (III) n'étant pas évidente à priori sans dégradation, seuls certains réactifs permettent d'effectuer cette transformation d'une manière satisfaisante. Il est nécessaire d'utiliser un réactif dur alkylant l'oxygène de préférence au carbone, tel qu'un dérivé du type Hal–Si<, Hal–B<, Hal–P<, $Hal-\overset{\overset{O}{\|}}{C}-OR'$, R' étant un alcoyle comportant 1 à 6 atomes de carbone, donnant lieu à la formation d'un dérivé O-silane, d'un borate, d'un phosphonate ou d'un carbonate, ou tel qu'un dérivé du type $MeS{-}SiMe_3$, $CH_3\,CO{-}ClO_4$, $Et_3\,Al$, Hal–Sn<.

Une étape suivante du procédé selon l'invention est essentiellement caractérisée en ce que le cétène de cétal (IV), une fois formé, est traité en milieu anhydre par un donneur de protons (qui attaque du côté le plus dégagé de la molécule et conduit ainsi à l'épimérisation souhaitée). La réussite de cette étape n'était pas non plus évidente à priori.

La réaction n'a pas lieu, en effet, d'une manière satisfaisante en présence de n'importe quel donneur de protons. Ceux-ci doivent être suffisamment volumineux pour attaquer la molécule par sa face la moins encombrée, donnant ainsi lieu à la formation du dérivé cis. La réaction doit être effectuée à basse température pour réaliser une protonation cinétique.

Bien entendu, les diverses réactions du procédé de l'invention doivent être effectuées en milieu anhydre: formation de l'énolate, attaque par le réactif Y-A, ou même attaque par le donneur de protons.

Finalement, la réussite du procédé de l'invention nécessite des conditions opératoires bien déterminées tant pour le choix du réactif O-alkylant que pour le choix du donneur de protons.

L'invention a également pour objet une variante du procédé précédent, essentiellement caractérisée en ce que l'on fait réagir directement le donneur de protons sur l'énolate (III) pour obtenir le dérivé cis désiré.

Des considérations analogues à celles développées pour le procédé précédent s'appliquent à cette variante. Seules des conditions opératoires bien déterminées permettent d'accéder à un pourcentage d'épimérisation intéressant.

Le procédé de l'invention permet ainsi d'obtenir des rendements en dérivés cis approchant 70%, ce qui est fort éloigné de l'équilibre thermodynamique précédemment indiqué. La transformation en ester d'alcoyle d'acide cischrysanthémique n'étant pas complète, l'acide cischrysanthémique recherché est isolé du mélange par des méthodes connues en soi. On peut par exemple saponifier le mélange d'esters pour obtenir le mélange des acides (1 S,trans) et (1 R,cis) correspondants. On insolubilise ensuite l'acide (1 S,trans)chrysanthémique par formation de son sel de L (+) thréo 1-p-nitrophényl-2-diméthylaminopropane-1,3-diol, élimine ce sel par filtration, acidifie le filtrat et recueille, après traitement, l'acide (1 R,cis)chrysanthémique désiré. Un exemple de cet isolement est donné plus loin dans la partie expérimentale.

Le procédé, objet de l'invention, a notamment pour intérêt de permettre la transformation de l'acide 1 S,transchrysanthémique en acide 1 R,cischrysanthémique, dans des conditions avantageuses, à l'aide de réactifs facilement accessibles.

Cette transformation trouve son utilité dans le fait que l'acide 1 S,transchrysanthémique provenant du dédoublement de l'acide dl cistranschrysanthémique obtenu par voie synthétique conduit, par oxydation, puis réaction de wittig sur l'aldéhyde résultant, aux acides (1 S,trans)-2,2-diméthyl-3-(2,2-dihalovinyl)cyclopropane-1-carboxyliques dont les esters ne présentent pas de propriétés insecticides intéressantes. L'acide (1 S,trans)chrysanthémique apparaît donc comme un résidu inutilisable du dédoublement de l'acide dl cistranschrysanthémique.

Au contraire, l'acide (1 R,cis)chrysanthémique conduit, par une suite de réactions analogues, aux acides (1 R,cis)-2,2-diméthyl-3-(2,2-dihalovinyl)cyclopropane-1-carboxyliques dont les esters sont doués de remarquables propriétés insecticides.

L'invention a donc pour objet un procédé d'épimérisation des esters d'alcoyle de l'acide transchrysanthémique, caractérisé en ce que l'on fait réagir, à basse température, en milieu anhydre, au sein d'un solvant, un ester d'alcoyle de l'acide transchrysanthémique de formule (II) :

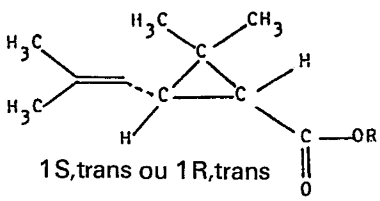

(II)

1 S,trans ou 1 R,trans

formule dans laquelle R représente un radical alcoyle comportant de 1 à 6 atomes de carbone, avec une base forte alcaline pour obtenir un composé de formule générale (III) :

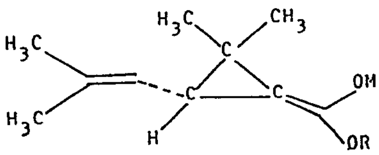

(III)

dans laquelle M représente un métal alcalin et R est défini comme précédemment, soumet le composé de formule générale (III) à l'action d'un réactif Y-A, formule dans laquelle A représente un atome de chlore, de brome ou d'iode ou un autre anion dérivé d'un hydracide et Y représente un radical organique tel que le réactif Y-A soit susceptible de former un cétène de cétal, pour obtenir un composé de formule (IV) :

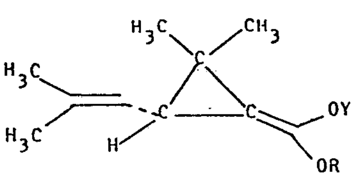

(IV)

formule dans laquelle Y et R conservent les significations précitées, soumet le composé (IV), à basse température, au sein d'un solvant en milieu anhydre, à l'action d'un donneur de protons, obtient un mélange d'ester d'alcoyle de l'acide cischrysanthémique et d'ester d'alcoyle de l'acide transchrysanthémique et sépare l'ester d'alcoyle de l'acide cischrysanthémique (I) de ce mélange puis isole ledit acide cis (I) par un procédé connu en soi.

L'invention a plus particulièrement pour objet un procédé d'épimérisation des esters d'alcoyle de l'acide 1 S,transchrysanthémique, caractérisé en ce que l'on part d'un ester d'alcoyle de l'acide 1 S,transchrysanthémique (II) et obtient l'acide 1 R,cischrysanthémique (I).

Dans les composés de formule (II), (III) et (IV), R représente un radical méthyle, éthyle, propyle linéaire ou ramifié, butyle linéaire ou ramifié, pentyle linéaire ou ramifié, hexyle linéaire ou ramifié. Dans le composé de formule (III), M représente un atome de lithium, de sodium ou de potassium. Dans le réactif Y-A, Y représente notamment un radical dialcoyloxyphosphonyle, borure de dialcoyle, borure d'éthylènedioxyle, diméthylterbutylsilyle ou triméthylsilyle.

L'invention a également pour objet une variante du procédé défini précédemment, caractérisée en ce que l'on soumet directement le composé (III), à basse température, au sein d'un solvant, en milieu anhydre, à l'action d'un donneur de protons, pour obtenir un mélange d'ester d'alcoyle de l'acide cischrysanthémique et d'ester d'alcoyle de l'acide transchrysanthémique, puis sépare l'ester d'alcoyle de l'acide cischrysanthémique, et isole l'acide cischrysanthémique par un procédé connu en soi, comme dans le procédé défini précédemment.

Dans des conditions préférentielles du procédé de l'invention ou de sa variante, le radical R est un radical méthyle ou éthyle, la base forte alcaline est le diisopropylamidure de lithium, la basse température à laquelle on effectue les réactions est comprise entre −20 et −70°C, le solvant au sein duquel on effectue les réactions est choisi dans le groupe constitué par les éthers, les hydrocarbures aromatiques et les mélanges de ces solvants.

D'une manière préférentielle, également dans le procédé de l'invention ou dans sa variante, le donneur de protons est choisi dans le groupe constitué par le 2,4-diméthyl-6-terbutylphénol, le chlorhydrate de collidine, le chlorhydrate d'acridine, le chlorhydrate de diisopropylamine, le chlorhydrate d'isopropyléthylamine, le chlorhydrate d'hexaméthylènetétramine, l'iodhydrate de collidine, la silice anhydre, un mélange de silice anhydre et d'acide chlorhydrique anhydre, l'acétylacétate d'éthyle, le chlorhydrate de 3,3,6,9,9-pentaméthyl-2,10-diazabicyclo-[4,4,0]-1-décène et l'eau, et le radical Y est choisi dans le groupe constitué par les radicaux dialcoyloxyphosphonyle, borure de dialcoyle, borure d'éthylènedioxyle, diméthylterbutylesilyle et triméthylsilyle.

L'invention a également pour objet, à titre de produits industriels nouveaux et notamment à titre de produits intermédiaires nécessaires à l'exécution du procédé de l'invention, les produits de formule (IV).

Les exemples suivants illustrent l'invention sans toutefois la limiter.

*Exemple 1*

*Acide 1R,cischrysanthémique au départ d'acide 1S,transchrysanthémate de méthyle*

*Stade A: Mélange de 1R,cischrysanthémate de méthyle et de 1S,transchrysanthémate de méthyle.*

Dans une solution de 1,2 $cm^3$ de diisopropylamine dans 5 $cm^3$ de tétrahydrofuran anhydre on introduit, à −10°C, 4,1 $cm^3$ de solution de butyllithium dans le cyclohexane, titrant 1,95 mol/$cm^3$, agite pendant 20 min à −10°C, ajoute à −50°C une solution de 1 g de 1S,transchrysanthémate de méthyle dans 5 $cm^3$ de tétrahydrofuran anhydre, agite à −50°C pendant 30 min, ajoute 3 $cm^3$ de chlorure de triméthylsilyle, agite à −50°C pendant 2½ h, ajoute 2,5 g de chlorhydrate de collidine, agite pendant 4 h à −5°C, laisse la température remonter lentement à 20°C, verse le mélange réactionnel dans une solution concentrée de phosphate monosodique, extrait à l'éther, sèche la phase organique, concentre à sec par distillation sous pression réduite, chormatographie le résidu sur silice en éluant par un mélange de benzène et de cyclohexane (50/50) et obtient 0,720 g de mélange de 1R,cischrysanthémate de méthyle et de 1S,transchrysanthémate de méthyle.

*Analyse par chromatographie en phase vapeur:*

Le mélange contient 56% de 1R,cischrysanthémate de méthyle, 42% de 1S,transchrysanthémate de méthyle et 2% d'impuretés de structure indéterminée (deux impuretés correspondant à 1,5% et 0,5% respectivement).

*Spectre de R.M.N.* (deutérochloroforme)

*a) Structure trans:*

– pics à 1,12-1,23 p.p.m. attribués aux hydrogènes des méthyles géminés,

– pic à 3,5 p.p.m. attribué aux hydrogènes du radical méthyle du méthoxycarbonyle,

– pics à 4,83-4,97 p.p.m. attribués à l'hydrogène de la double liaison éthylénique.

*b) Structure cis:*

– pics à 1,18-1,22 p.p.m. attribués aux hydrogènes des méthyles géminés,

– pic à 3,5 p.p.m. attribué aux hydrogènes du radical méthyle du méthoxycarbonyle,

– pics à 5,35-5,48 p.p.m. attribués à l'hydrogène de la double liaison éthylénique.

*Stade B: Saponification du mélange d'esters.*

On porte au reflux pendant 15 min sous azote, afin de la dégazer, une solution de 100 $cm^3$ de méthanol et de 60 $cm^3$ de lessive de soude (correspondant à 24 g de NaOH).

On introduit 0,720 g de mélange d'ester d'acide (1S,trans) et d'ester d'acide (1R,cis) obtenus au stade précédent dans 1,2 $cm^3$ de la solution de soude préalablement dégazée, porte le mélange réactionnel au reflux sous atmosphère inerte, maintient le reflux pendant 3 h, refroidit, ajoute 0,7 $cm^3$ d'eau, élimine le méthanol par distillation sous pression réduite, acidifie à pH=2, par addition d'une solution aqueuse d'acide chlorhydrique en maintenant la température au-dessous de 25°C, extrait au chlorure de méthylène, élimine le solvant par distillation sous pression réduite, obtient 0,657 g d'un mélange d'acide (1S,trans)-chrysanthémique et d'acide (1R,cis)chrysanthémique (43/57) utilisé tel quel pour le stade suivant.

*Stade C: Séparation de l'acide 1R,cischrysanthémique.*

On dissout 0,657 g de mélange d'acide (1S,trans) et (1R,cis)chrysanthémique précédemment obtenus dans un mélange de 0,3 $cm^3$ de méthanol et de 1,8 $cm^3$ d'éther isopropylique, ajoute progressivement, en agitant, 0,880 g de L (+) thréo-1-p-nitrophényl-2-diméthylamino-propane-1,3-diol, porte le mélange réactionnel au reflux, en 15 min sous atmosphère inerte, maintient le reflux pendant 15 min, refroidit à 0°C, agite 2 h à cette température, élimine par filtration le sel de L (+) thréo-1-p-n-nitrophényl-2-diméthylaminopropane-1,3-diol de l'acide (1S,trans)chrysanthémique qui s'est insolubilisé, lave ce précipité par un mélange glacé d'éther isopropylique et de méthanol (85/15), réunit liqueurs mères et lavages, ajoute lentement sans dépasser 25°C, jusqu'à acidité du rouge congo, une solution aqueuse d'acide chlorhydrique titrant 0,5 mol/l, décante, extrait la phase aqueuse à l'éther isopropylique, lave les solutions organiques à l'eau, concentre à sec par distillation sous pression réduite et obtient 0,308 g d'acide (1R,cis)chrysanthémique.

*Exemple 2*

*Mélange de 1R,cischrysanthémate de méthyle et de 1S,transchrysanthémate de méthyle*

Dans une solution de 1 $cm^3$ de diisopropylamine dans 5 $cm^3$ d'éther anhydre on introduit, à −10°C, 3,55 $cm^3$ de solution de butyllithium dans le cyclohexane, titrant 1,85 mmol/$cm^3$, agite à −10°C pendant 20 min, introduit à −40°C une solution de 1 g de 1S,transchrysanthémate de méthyle dans 5 $cm^3$ d'éther, agite pendant 1 h à −40°C, ajoute 2 $cm^3$ de chlorure de triméthylsilyle, agite pendant 2½ h à −40°C, ajoute 2,5 g de chlorhydrate de collidine, agite à −40°C pendant 4 h, laisse remonter lentement la température à 20°C, verse le mélange réactionnel sur une solution saturée de phosphate monosodique, extrait à l'éther, concentre à sec par distillation sous pression réduite, chromatographie le résidu sur silice en éluant par un mélange de benzène et de cyclohexane (50/50) et obtient 0,581 g de mélange de 1R,cischrysanthémate de méthyle et de 1S,transchrysanthémate de méthyle.

*Analyse par chromatographie en phase vapeur:*

Le mélange contient 74,7% de 1R,transchrysanthémate de méthyle et 25,3% de 1S,transchrysanthémate de méthyle.

*Exemple 3*

*Mélange de 1R,cischrysanthémate de méthyle et de 1S,transchrysanthémate de méthyle*

Dans une solution de 1 $cm^3$ de diisopropylamine dans 5 $cm^3$ de tétrahydrofuran anhydre on introduit, à −10°C, 3,85 $cm^3$ de solution de butyllithium dans le cyclohexane, titrant 1,7 mmol/$cm^3$, agite à −10°C pendant 20 min, ajoute à −40°C une solution de 1 g de 1S,transchrysanthémate de méthyle dans 5 $cm^3$ de tétrahydrofuran, agite pendant 1 h à −40°C, ajoute 3 $cm^3$ de chlorure de trinéthylsilyle, agite à −40°C pendant 2½ h, ajoute 2,5 g de chlorhydrate de 3,3,6,9,9-pentaméthyl-2,10-diazabicyclo-[4,4,0]-1-décène, agite pendant 4 h à −40°C, laisse la température remonter lentement à 20°C, verse le mélange réactionnel dans une solution saturée de phosphate monosodique, extrait à l'éther, concentre à sec par distillation sous pression réduite, chromatographie le résidu sur silice en éluant avec un mélange de benzène et de cyclohexane (50/50) et obtient 0,775 g d'un mélange de 1R,cischrysanthémate de méthyle et de 1S,transchrysanthémate de méthyle.

*Analyse par chromatographie en phase vapeur:*

Le mélange contient 63% de 1R,cischrysanthémate de méthyle et 37% de 1S,transchrysanthémate de méthyle.

*Exemple 4*

*Mélange de 1R,cischrysanthémate de méthyle et de 1S,transchrysanthémate de méthyle*

Dans une solution de 1,2 $cm^3$ de diisopropylamine dans 5 $cm^3$ d'éther anhydre on introduit, à −10°C, 4,45 $cm^3$ de solution de butyllithium dans le cyclohexane titrant 1,8 mmol/$cm^3$, agite pendant 15 min à −10°C, ajoute à −50°C une solution de 1 g de 1S,transchrysanthémate de méthyle dans 5 $cm^3$ d'éther anhydre, agite à −50°C pendant 30 min, ajoute 3 $cm^3$ de chlorure de triméthylsilyle, agite pendant 2½ h à −50°C, ajoute 2,5 g de chlorhydrate de collidine, agite pendant 3 h à −50°C, laisse la température remonter lentement à 20°C, verse le mélange réactionnel dans une solution saturée de phosphate monosodique, extrait à l'éther, concentre la solution organique à sec par distillation sous pression réduite, chromatographie le résidu sur silice en éluant par un mélange de benzène et de cyclohexane (50/50) et obtient 0,760 g d'un mélange de 1R,cischrysanthémate de méthyle et de 1S,transchrysanthémate de méthyle.

*Analyse par chromatographie en phase vapeur:*

Le mélange contient 63% de 1R,cischrysanthémate de méthyle et 37% de 1S,transchrysanthémate de méthyle.

*Exemple 5*

*Mélange de 1R,cischrysanthémate de méthyle et de 1S,transchrysanthémate de méthyle*

Dans une solution de 1,2 $cm^3$ de diisopropylamine dans 5 $cm^3$ d'éther anhydre on introduit, à −10°C, 4,1 $cm^3$ d'une solution de butyllithium dans le cyclohexane titrant 1,95 mmol/$cm^3$, agite pendant 20 min à −10°C, introduit à −40°C une solution de 1 g de 1S,transchrysanthémate de méthyle dans 5 $cm^3$ d'éther, agite pendant 30 min à −40°C, ajoute 3 $cm^3$ de chlorure de triméthylsilyle, agite pendant 2½ h à −40°C, ajoute 2,5 g de chlorhydrate de collidine, agite pendant 15 h à −30°C, verse le mélange réactionnel dans une solution saturée de phosphate monosodique, extrait l'éther, concentre la solution organique à sec par distillation sous pression réduite, chromatographie le résidu sur silice en éluant avec un mélange de benzène et de cyclohexane (50/50) et obtient 0,600 g de mélange 1R,cischrysanthémate de méthyle et de 1S,transchrysanthémate de méthyle.

*Analyse par chromatographie en phase vapeur:*

Le mélange contient 78,1% de 1R,cischrysanthémate de méthyle et 21,9% de 1S,transchrysanthémate de méthyle.

*Exemple 6*

*Mélange de 1R,cischrysanthémate de méthyle et de 1S,transchrysanthémate de méthyle*

Dans une solution de 1,2 $cm^3$ de diisopropylamine dans 5 $cm^3$ de tétrahydrofuran anhydre on introduit, à −10°C, 4,7 $cm^3$ d'une solution de butyllithium dans le cyclohexane titrant 1,7 mmol/$cm^3$, agite pendant 30 min à −10°C, ajoute à −40°C une solution de 1 g de 1S,transchrysanthémate de méthyle dans 5 $cm^3$ de tétrahydrofuran, agite pendant 30 min à −40°C, ajoute 3 $cm^3$ de chlorure de triméthylsilyle, agite pendant 2 h à −40°C, verse le mélange réactionnel sur de la glace, extrait à l'éther, concentre la solution organique à sec par distillation sous pression réduite, chromatographie le résidu sur silice en éluant par un mélange de benzène et de cyclohexane (50/50) et obtient 0,852 g de mélange de 1R,cischrysanthémate de méthyle et de 1S,transchrysanthémate de méthyle.

*Analyse par chromatographie en phase vapeur:*

Le mélange contient 56% de 1R,cischrysanthémate de méthyle et 44% de 1S,transchrysanthémate de méthyle.

*Exemple 7*

*Mélange de 1R,cischrysanthémate de méthyle et de 1S,transchrysanthémate de méthyle*

On opère de façon analogue à celle de l'exemple 6, mais après 2 h d'agitation à −40°C on introduit 1,6 g de chlorhydrate de collidine, agite à −40°C pendant 60 h, verse le mélange réactionnel dans une solution saturée de phosphate monosodique, extrait l'éther, concentre la solution organique à sec par distillation sous pression réduite, chromatographie le résidu sur silice en éluant par un mélange de benzène et de cyclohexane (50/50) et obtient 0,569 g de mélange 1R,cischrysanthémate de méthyle et de 1S,transchrysanthémate de méthyle.

*Analyse par chromatographie en phase vapeur:*

Le mélange contient 69% de 1R,cischrysanthémate de méthyle et 31% de 1S,transchrysanthémate de méthyle.

*Exemple 8*

*Mélange de 1R,cischrysanthémate de méthyle et de 1S,transchrysanthémate de méthyle*

Dans un mélange de 0,6 $cm^3$ de diisopropylamine dans 5 $cm^3$ de tétrahydrofuran anhydre on introduit, à $-10°C$, 2,35 $cm^3$ de solution de butyllithium dans le cyclohexane titrant 1,7 mmol/$cm^3$, agite pendant 30 min à $-10°C$, ajoute à $-40°C$ une solution de 0,5 g de 1S,transchrysanthémate de méthyle dans 5 $cm^3$ de tétrahydrofuran, agite pendant ½ h à $-40°C$, ajoute 2,8 g de chlorhydrate de 3,3,6,9,9-pentaméthyl-2,10-diazabicyclo-[4,4,0]-1-décène, agite pendant 60 h à $-40°C$, verse le mélange réactionnel dans une solution saturée de phosphate monosodique, extrait à l'éther, concentre la solution organique à sec par distillation sous pression réduite, chromatographie le résidu sur silice en éluant par un mélange de benzène et de cyclohexane (50/50) et obtient 0,419 g de mélange de 1R,cischrysanthémate de méthyle et de 1S,transchrysanthémate de méthyle.

*Analyse par chromatographie en phase vapeur:*

Le mélange contient 45% de 1R,cischrysanthémate de méthyle et 55% de 1S,transchrysanthémate de méthyle.

*Exemple 9*

*Mélange de 1R,cischrysanthémate de méthyle et de 1S,transchrysanthémate de méthyle*

Dans une solution de 0,8 $cm^3$ de diisopropylamine dans 3,5 $cm^3$ de tétrahydrofuran anhydre on introduit, à $-10°C$, 3,65 $cm^3$ de solution de butyllithium dans le cyclohexane titrant 1,5 mmol/$cm^3$, agite pendant 15 min à $-10°C$, ajoute à $-20°C$ une solution de 1 g de 1S,transchrysanthémate de méthylène dans 5 $cm^3$ de tétrahydrofuran, agite pendant 3 h à $-20°C$, ajoute 2,8 g de chlorhydrate de 3,3,6,9,9-pentaméthyl-2,10-diazabicyclo-[4,4,0]-1-décène, agite pendant 40 h à $-20°C$, verse le mélange réactionnel dans une solution saturée de phosphate monosodique, extrait à l'éther, concentre la solution organique à sec par distillation sous pression réduite, chromatographie le résidu sur silice en éluant avec un mélange de benzène et de cyclohexane (50/50) et obtient 0,778 g d'un mélange de 55% de 1R,cischrysanthémate de méthyle et de 45% de 1S,transchrysanthéate de méthyle.

*Exemple 10*

*Mélange de 1R,cischrysanthémate de méthyle et de 1S,transchrysanthémate de méthyle*

Dans une solution de 0,56 $cm^3$ de diisopropylamine dans 6 $cm^3$ de tétrahydrofuran anhydre on introduit, à $-10°C$, 2,15 $cm^3$ de solution de butyllithium dans le cyclohexane, titrant 1,9 mmol/$cm^3$, agite pendant 20 min à $-10°C$, ajoute à $-20°C$ une solution de 0,5 g de 1S,transchrysanthémate de méthyle dans 5 $cm^3$ de tétrahydrofuran, agite à $-20°C$ pendant 1½ h, verse dans une solution de 1,35 g de 2,6-terbutyl-4-méthylphénol dans 10 $cm^3$ de tétrahydrofuran anhydre, refroidie préalablement à $-20°C$, agite pendant 20 h à $-20°C$, verse le mélange réactionnel dans une solution aqueuse saturée de phosphate monosodique, extrait à l'éther, concentre la solution organique à sec par distillation sous pression réduite, chromatographie le résidu sur silice en éluant par un mélange de benzène et de cyclohexane (50/50) et obtient 0,463 g de mélange de 1R,cischrysanthémate de méthyle et de 1S,transchrysanthémate de méthyle.

*Analyse par chromatographie en phase vapeur:*

Le mélange contient 42% de 1R,cischrysanthémate de méthyle et 58% de 1S,transchrysanthémate de méthyle.

*Exemple 11*

*Mélange de 1R,cischrysanthémate de méthyle et de 1S,transchrysanthémate de méthyle*

Dans une solution de 0,56 $cm^3$ de diisopropylamine dans 6 $cm^3$ de tétrahydrofuran anhydre on introduit, à $-10°C$, 2,15 $cm^3$ de solution de butyllithium dans le cyclohexane, titrant 19 mmol/$cm^3$, agite à $-10°C$ pendant 20 min, ajoute à $-20°C$ une solution de 0,5 g de 1S,transchrysanthémate de méthyle dans 5 $cm^3$ de tétrahydrofuran, agite à $-20°C$ pendant 1½ h, verse dans une suspension de 1,1 g de chlorhydrate de diisopropyléthylamine dans 10 $cm^3$ de tétrahydrofuran anhydre, agite à $-20°C$ pendant 20 h, verse le mélange réactionnel dans une solution aqueuse saturée de phosphate monosodique, extrait à l'éther, concentre la solution organique à sec par distillation sous pression réduite, chromatographie le résidu sur silice en éluant par un mélange de benzène et de cyclohexane (50/50) et obtient 0,373 mg de mélange de 1R,cischrysanthémate de méthyle et de 1S,transchrysanthémate de méthyle.

*Analyse par chromatographie en phase vapeur:*

Le mélange contient 40% de 1R,cischrysanthémate de méthyle et 60% de 1S,transchrysanthémate de méthyle.

*Exemple 12*

*Mélange de 1R,cischrysanthémate de méthyle et de 1S,transchrysanthémate de méthyle*

Dans une solution de 0,77 $cm^3$ de diisopropylamine dans 7 $cm^3$ de tétrahydrofuran anhydre on introduit, à $-15°C$, 3,05 $cm^3$ de solution de butyllithium dans le cyclohexane titrant 1,8 mmol/$cm^3$, agite à $-15°C$ pendant 15 min, ajoute à $-25°C$ une solution de 1 g de 1S,transchrysanthémate de méthyle dans 10 $cm^3$ de tétrahydrofuran, agite pendant 3 h à $-20°C$, ajoute 0,954 g de chlorhydrate de collidine, agite à $-20°C$ pendant 18 h, verse le mélange réactionnel dans une solution aqueuse saturée de phosphate monosodique, extrait à l'éther, concentre la solution organique à

sec par distillation sous pression réduite, chromatographie le résidu sur silice en éluant par un mélange de benzène et de cyclohexane (50/50) et obtient 0,473 g de mélange de 1R,cischrysanthémate de méthyle et de 1S,transchrysanthémate de méthyle.

*Analyse par chromatographie en phase vapeur:*

Le mélange contient 51% de 1R,cischrysanthémate de méthyle et 49% de 1S,transchrysanthémate de méthyle.

*Exemple 13*

*Mélange de 1R,cischrysanthémate de méthyle et de 1S,transchrysanthémate de méthyle*

Dans une solution de 0,56 $cm^3$ de diisopropylamine dans 6 $cm^3$ de tétrahydrofuran anhydre on introduit, à −10°C, 2,15 $cm^3$ de solution de butyllithium dans le cyclohexane titrant 1,9 mmol/$cm^3$, agite à −10°C pendant 15 min, introduit à −20°C une solution de 0,5 g de 1S,transchrysanthémate de méthyle dans 5 $cm^3$ de tétrahydrofuran, agite pendant 1½ h à −20°C, verse dans une suspension de 1,4 g de chlorhydrate d'acridine dans 10 $cm^3$ de tétrahydrofuran anhydre refroidie préalablement à −20°C, agite pendant 60 h à −20°C, verse le mélange réactionnel dans une solution aqueuse saturée de phosphate monosodique, extrait l'éther, concentre à sec la solution organique par distillation sous pression réduite, chromatographie le résidu sur silice en éluant avec un mélange de benzène et de cyclohexane (50/50) et obtient 0,407 g de mélange 1R,cischrysanthémate de méthyle et de 1S,transchrysanthémate de méthyle.

*Analyse par chromatographie en phase vapeur:*

Le mélange contient 37% de 1R,cischrysanthémate de méthyle et 63% de 1S,transchrysanthémate de méthyle.

*Exemple 14*

*Mélange de 1R,cischrysanthémate de méthyle et de 1S,transchrysanthémate de méthyle*

A 0,6 $cm^3$ de diisopropylamine dans 2,5 $cm^3$ de tétrahydrofuran anhydre, on ajoute goutte à goutte, à −10°C sous atmosphère d'azote, 2,5 $cm^3$ d'une solution 1,6 mol/l de BuLi dans le cyclohexane. On agite ensuite 15 min à cette température puis refroidit la solution à −40°C, ajoute goutte à goutte 500 mg de 1R,transchrysanthémate de méthyle dans 5 $cm^3$ de tétrahydrofuran et agite à cette température pendant ¾ h. On ajoute goutte à goutte 750 mg de chlorure de diméthylterbutylsilyle dans 5 $cm^3$ de tétrahydrofuran anhydre et poursuit l'agitation à −40°C pendant 2 h.

A) On prélève 5 $cm^3$ de cette solution que l'on verse dans l'eau glacée, on extrait à l'éther, sèche la solution organique, concentre sous pression réduite, chromatographie sur silice (éluant: benzène/cyclohexane 1/1) et obtient 50 mg de mélange contenant 36% d'isomère cis et 64% d'isomère trans.

B) A la solution restante on ajoute 900 mg de chlorhydrate de collidine et agite une nuit à −40°C. On verse la solution dans une solution saturée de phosphate monosodique, extrait à l'éther, sèche et évapore le solvant sous pression réduite. On chromatographie sur silice (éluant: benzène/cyclohexane 1/1). On obtient 175 mg de mélange contenant 48% d'isomère cis et 52% d'isomère trans.

*Exemple 15*

*Mélange de 1R,cischrysanthémate de méthyle et de 1S,transchrysanthémate de méthyle*

A une solution de 1,2 $cm^3$ de diisopropylamine dans 5 $cm^3$ de tétrahydrofuran anhydre, refroidie à −10°C, on ajoute goutte à goutte, sous atmosphère d'azote, 4,1 $cm^3$ d'une solution 1,95 mol/l de BuLi dans le cyclohexane puis agite 1 h à −10°C.

On refroidit à −40°C et ajoute une solution de 1 g de 1S,transchrysanthémate de méthyle dans 5 $cm^3$ de tétrahydrofuran anhydre et continue à agiter pendant ½ h à −40°C. Après la fin de l'addition, on ajoute 1 g de 1-chloro-2,5-dioxaborolane dans 5 $cm^3$ de tétrahydrofuran anhydre puis agite encore pendant 2 h à cette température.

A) On prélève 5 $cm^3$ de la solution précédente que l'on verse sur de la glace et on extrait à l'éther; on sèche la phase organique et évapore le solvant sous pression réduite. On obtient 443 mg d'huile brute contenant 53,1% d'isomère cis et 46,9% d'isomère trans.

B) A la solution restante, on ajoute 2 g de chlorhydrate de collidine et on poursuit l'agitation pendant 20 h à −40°C. On verse dans une solution saturée de phosphate monosodique, extrait à l'éther, sèche la phase organique et évapore le solvant sous pression réduite. On obtient 2,07 g d'huile brute contenant 50,6% d'isomère cis et 49,4% d'isomère trans.

*Exemple 16*

*Mélange de 1R,cischrysanthémate de méthyle et de 1S,transchrysanthémate de méthyle*

A une solution de 1,2 $cm^3$ de diisopropylamine anhydre dans 5 $cm^3$ de tétrahydrofuran anhydre refroidie à −15°C, on ajoute goutte à goutte, sous atmosphère d'azote, 4,7 $cm^3$ d'une solution de 1,7 mol/l de BuLi dans le cyclohexane. On agite pendant 1 h à cette température, on refroidit à −40°C et on ajoute goutte à goutte une solution de 1 g de 1S,transchrysanthémate de méthyle dans 5 $cm^3$ de tétrahydrofuran anhydre et poursuit l'agitation à cette température pendant ½ h après la fin de l'addition. On ajoute 1,5 $cm^3$ de diméthoxychloroborane à −40°C et agite pendant 1 h à cette température.

A) On prélève 3 $cm^3$ de solution que l'on verse sur de la glace, extrait à l'éther, sèche la phase organique et concentre sous pression réduite, on obtient 162 mg d'huile brute contenant 53,3% d'isomère cis et 46,7% d'isomère trans.

B) A la solution restante, on ajoute 2 g de chlorhydrate de collidine et poursuit l'agitation à −40°C pendant 16 h, on verse dans une solution de phosphate monosodique concentrée et extrait

à l'éther, sèche la phase organique et évapore sous pression réduite. On obtient 830 mg d'huile brute contenant 53,5% d'isomère cis et 46,5% d'isomère trans.

*Exemple 17*

*Mélange de 1R,cischrysanthémate de méthyle et de 1S,transchrysanthémate de méthyle*

A une solution de 1,2 $cm^3$ de diisopropylamine anhydre dans 5 $cm^3$ de tétrahydrofuran anhydre, refroidie à −10°C, on ajoute goutte à goutte, sous atmosphère d'azote, 4,7 $cm^3$ d'une solution 1,7 mol/l de BuLi dans le cyclohexane puis on poursuit l'agitation pendant ½ h. On refroidit la solution à −40°C et ajoute goutte à goutte 1 g de 1S,transchrysanthémate de méthyle dans 5 $cm^3$ de tétrahydrofuran anhydre et continue à agiter à −40°C pendant ½ h. On ajoute ensuite goutte à goutte, à −40°C, une solution de 1,72 g de chlorophosphonate de diéthyle dans 5 $cm^3$ de tétrahydrofuran et continue l'agitation à −40°C pendant 2 h.

A) On prélève 5 $cm^3$ de cette solution et la verse dans de l'eau glacée, on extrait à l'éther, sèche la phase organique et évapore le solvant sous pression réduite. On obtient 360 mg d'huile brute contenant 38% d'isomère cis et 62% d'isomère trans.

B) A la solution restante, on ajoute 1,5 g de chlorhydrate de collidine et agite pendant 16 h à −40°C, on verse dans une solution saturée de phosphate monosodique, extrait à l'éther, sèche la phase éthérée et évapore le solvant sous pression réduite. On obtient 951 mg d'huile brute contenant 43% d'isomère cis et 57% d'isomère trans.

**Revendications** pour les Etats contractants: BE, CH, DE, GB, IT, LI, LU, NL, SE

1. Procédé d'épimérisation des esters d'alcoyle de l'acide transchrysanthémique, caractérisé en ce que l'on fait réagir, à basse température, en milieu anhydre, au sein d'un solvant, un ester d'alcoyle de l'acide transchrysanthémique de formule (II)

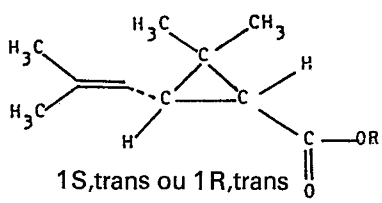

(II)

1S,trans ou 1R,trans

formule dans laquelle R représente un radical alcoyle comportant de 1 à 6 atomes de carbone, avec une base forte alcaline pour obtenir un composé de formule générale (III)

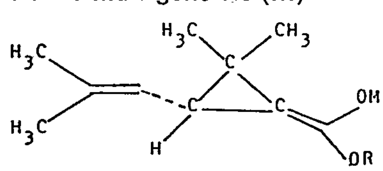

(III)

dans laquelle M représente un métal alcalin et R est défini comme précédemment, soumet le composé de formule générale (III) à l'action d'un réactif Y-A, formule dans laquelle A représente un atome de chlore, de brome ou d'iode ou un autre anion dérivé d'un hydracide et Y représente un radical organique tel que le réactif Y-A soit susceptible de former un cétène de cétal, pour obtenir un composé de formule (IV)

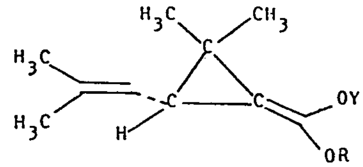

(IV)

formule dans laquelle Y et R conservent les significations précitées, soumet le composé (IV), à basse température, au sein d'un solvant en milieu anhydre, à l'action d'un donneur de protons, obtient un mélange d'ester d'alcoyle de l'acide cischrysanthémique et d'ester d'alcoyle de l'acide transchrysanthémique et enfin sépare l'ester d'alcoyle de l'acide chrysanthémique (I)

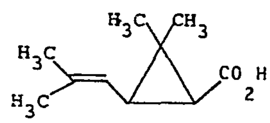

(I)

1R,cis ou 1S,cis

de ce mélange, puis isole ledit acide cis (I) par un procédé connu en soi.

2. Procédé selon la revendication 1 d'épimérisation des esters d'alcoyle de l'acide 1S,transchrysanthémique, caractérisé en ce que l'on part d'un ester d'alcoyle de l'acide 1S,transchrysanthémique (II) et obtient l'acide 1R,cischrysanthémique (I).

3. Variante du procédé selon la revendication 1, caractérisée en ce que l'on soumet directement le composé (III), à basse température, au sein d'un solvant, en milieu anhydre, à l'action d'un donneur de protons pour obtenir un mélange d'ester d'alcoyle de l'acide cischrysanthémique et isole l'acide cischrysanthémique par un procédé connu en soi, comme dans le procédé de la revendication 1.

4. Procédé selon l'une des revendications 1, 2 ou 3, caractérisé en ce que le radical R est un radical méthyle ou éthyle.

5. Procédé selon l'une des revendications 1 à 4, caractérisé en ce que la base forte alcaline est le diisopropylamidure de lithium.

6. Procédé selon l'une des revendications 1 à 5, caractérisé en ce que la basse température à laquelle on effectue les réactions est comprise entre −20 et −70°C.

7. Procédé selon l'une des revendications 1 à 6, caractérisé en ce que le solvant au sein duquel on effectue les réactions est choisi dans le groupe constitué par les éthers, les hydrocarbures aromatiques et les mélanges de ces solvants.

8. Procédé selon l'une des revendications 1 à 7, caractérisé en ce que le donneur de protons est choisi dans le groupe constitué par le 2,4-diméthyl-6-terbutylphénol, le chlorhydrate de collidine, le chlorhydrate d'acridine, le chlorhydrate de

diisopropylamine, le chlorhydrate d'isopropyléthylamine, le chlorhydrate d'hexaméthylènetétramine, l'iodhydrate de collidine, la silice anhydre, un mélange de silice anhydre et d'acide chlorhydrique anhydre, l'acétylacétate d'éthyle, le chlorhydrate de 3,3,6,9,9-pentaméthyl-2,10-diazabicyclo-[4,4,0]-1-décène et l'eau.

9. Procédé selon l'une des revendications 1, 2 ou 4 à 8, caractérisé en ce que le radical Y est choisi dans le groupe constitué par les radicaux dialcoyloxyphosphonyle, borure de dialcoyle, borure d'éthylènedioxyle, diméthylterbutylsilyle et triméthylsilyle.

10. A titre de produits industriels nouveaux, les produits de formule (IV), tels que définis à la revendication 1.

**Revendications** pour l'Etat contractant: AT

1. Procédé d'épimérisation des esters d'alcoyle de l'acide transchrysanthémique, caractérisé en ce que l'on fait réagir, à basse température, en milieu anhydre, au sein d'un solvant, un ester d'alcoyle de l'acide transchrysanthémique de formule (II)

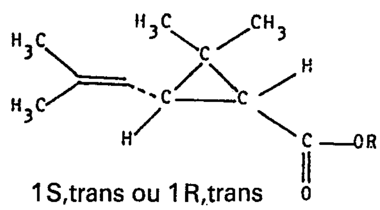

(II)

1S,trans ou 1R,trans

formule dans laquelle R représente un radical alcoyle comportant de 1 à 6 atomes de carbone, avec une base forte alcaline pour obtenir un composé de formule générale (III)

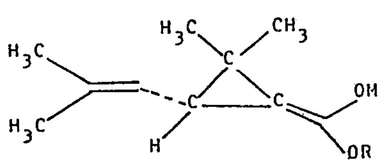

(III)

dans laquelle M représente un métal alcalin, et R est défini comme précédemment, soumet le composé de formule générale (III) à l'action d'un réactif Y-A, formule dans laquelle A représente un atome de chlore, de brome ou d'iode ou un autre anion dérivé d'un hydracide et Y représente un radical organique tel que le réactif Y-A soit susceptible de former un cétène de cétal, pour obtenir un composé de formule (IV)

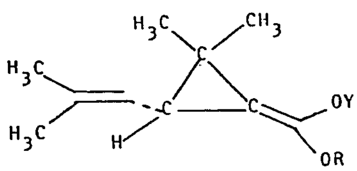

(IV)

formule dans laquelle Y et R conservent les significations précitées, soumet le composé (IV), à basse température, au sein d'un solvant en milieu anhydre, à l'action d'un donneur de protons, obtient un mélange d'ester d'alcoyle de l'acide cischrysanthémique et d'ester d'alcoyle de l'acide transchrysanthémique et enfin sépare l'ester d'alcoyle de l'acide cischrysanthémique (I)

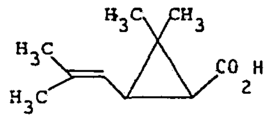

(I)

1R,cis ou 1S,cis

de ce mélange, puis isole ledit acide cis (I) par un procédé connu en soi.

2. Procédé selon la revendication 1 d'épimérisation des esters d'alcoyle de l'acide 1S,transchrysanthémique, caractérisé en ce que l'on part d'un ester d'alcoyle de l'acide 1S,transchrysanthémique (II) et obtient l'acide 1R,cischrysanthémique (I).

3. Variante du procédé selon la revendication 1, caractérisée en ce que l'on soumet directement le composé (III), à basse température, au sein d'un solvant, en milieu anhydre, à l'action d'un donneur de protons pour obtenir un mélange d'ester d'alcoyle de l'acide cischrysanthémique et d'ester d'alcoyle de l'acide transchrysanthémique, puis sépare l'ester d'alcoyle de l'acide cischrysanthémique et isole l'acide cischrysanthémique par un procédé connu en soi, comme dans le procédé de la revendication 1.

4. Procédé selon l'une des revendications 1, 2 ou 3, caractérisé en ce que le radical R est un radical méthyle ou éthyle.

5. Procédé selon l'une des revendications 1 à 4, caractérisé en ce que la base forte alcaline est le diisopropylamidure de lithium.

6. Procédé selon l'une des revendications 1 à 5, caractérisé en ce que la basse température à laquelle on effectue les réactions est comprise entre −20 et −70°C.

7. Procédé selon l'une des revendications 1 à 6, caractérisé en ce que le solvant au sein duquel on effectue les réactions est choisi dans le groupe constitué par les éthers, les hydrocarbures aromatiques et les mélanges de ces solvants.

8. Procédé selon l'une des revendications 1 à 7, caractérisé en ce que le donneur de protons est choisi dans le groupe constitué par le 2,4-diméthyl-6-terbutylphénol, le chlorhydrate de collidine, le chlorhydrate d'acridine, le chlorhydrate de diisopropylamine, le chlorhydrate d'isopropyléthylamine, le chlorhydrate d'hexaméthylènetétramine, l'iodhydrate de collidine, la silice anhydre, un mélange de silice anhydre et d'acide chlorhydrique anhydre, l'acétylacétate d'éthyle, le chlorhydrate de 3,3,6,9,9-pentaméthyl-2,10-diazabicyclo-[4,4,0]-1-décène et l'eau.

9. Procédé selon l'une des revendications 1, 2 ou 4 à 8, caractérisé en ce que le radical Y est choisi dans le groupe constitué par les radicaux dialcoyloxyphosphonyle, borure de dialcoyle, borure d'éthylènedioxyle, diméthylterbutylsilyle et triméthylsilyle.

**Patentansprüche** für die Vertragsstaaten: BE, CH, DE, GB, IT, LI, LU, NL, SE

1. Verfahren zur Epimerisierung von Alkylestern der trans-Chrysanthemumsäure, dadurch gekennzeichnet, dass man bei niedriger Temperatur in wasserfreiem Milieu in dem Medium eines Lösungsmittels einen Alkylester der trans-Chrysanthemumsäure der Formel (II)

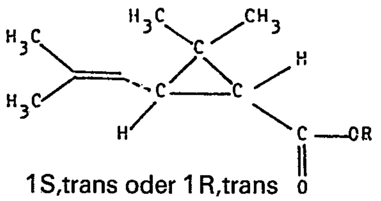

(II)

1S,trans oder 1R,trans

worin R einen Alkylester mit 1 bis 6 Kohlenstoffatomen bedeutet, mit einer starken Alkalibase umsetzt, um eine Verbindung der allgemeinen Formel (III)

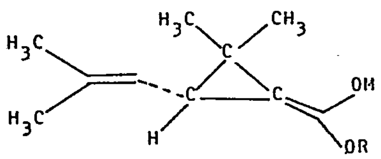

(III)

zu erhalten, worin M ein Alkalimetall bedeutet und R wie vorstehend definiert ist, die Verbindung der allgemeinen Formel (III) der Einwirkung eines Reagens Y-A unterzieht, in dem A ein Chlor-, Brom- oder Jodatom oder ein anderes, sich von einer Wasserstoffsäure ableitendes Anion bedeutet und Y einen organischen Rest darstellt, derart, dass das Reagens Y-A befähigt ist, ein Ketalketen zu bilden, um eine Verbindung der Formel (IV)

(IV)

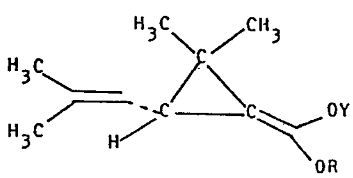

zu erhalten, worin Y und R wie vorstehend definiert sind, die Verbindung (IV) bei niedriger Temperatur in dem Medium eines Lösungsmittels in wasserfreiem Milieu der Einwirkung eines Protonendonators unterzieht, eine Mischung des Alkylesters der cis-Chrysanthemumsäure und des Alkylesters der trans-Chrysanthemumsäure erhält, und schliesslich den Alkylester der cis-Chrysanthemumsäure (I)

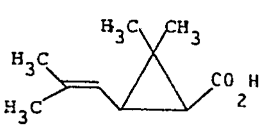

(I)

1R,cis oder 1S,cis

aus diesem Gemisch abtrennt und dann die cis-Säure (I) mit Hilfe eines an sich bekannten Verfahrens isoliert.

2. Verfahren gemäss Anspruch 1 zur Epimerisierung von Alkylestern der 1S,trans-Chrysanthemumsäure, dadurch gekennzeichnet, dass man von einem Alkylester der 1S,trans-Chrysanthemumsäure (II) ausgeht und die 1R,cis-Chrysanthemumsäure (I) gewinnt.

3. Variante des Verfahrens gemäss Anspruch 1, dadurch gekennzeichnet, dass man direkt die Verbindung (III) bei niedriger Temperatur in dem Medium eines Lösungsmittels in wasserfreiem Milieu der Einwirkung eines Protonendonators unterzieht, um eine Mischung des Alkylesters der cis-Chrysanthemumsäure und des Alkylesters der trans-Chrysanthemumsäure zu erhalten, danach den Alkylester der cis-Chrysanthemumsäure abtrennt und die cis-Chrysanthemumsäure mit Hilfe eines an sich bekannten Verfahrens, wie bei dem Verfahren gemäss Anspruch 1, isoliert.

4. Verfahren gemäss einem der Ansprüche 1, 2 oder 3, dadurch gekennzeichnet, dass der Rest R einen Methyl- oder Ethylrest bedeutet.

5. Verfahren gemäss einem der Ansprüche 1 bis 4, dadurch gekennzeichnet, dass die starke Alkalibase Lithiumdiisopropylamid ist.

6. Verfahren gemäss einem der Ansprüche 1 bis 5, dadurch gekennzeichnet, dass die nidriege Temperatur, bei der man die Reaktionen durchführt, zwischen −20 und −70°C liegt.

7. Verfahren gemäss einem der Ansprüche 1 bis 6, dadurch gekennzeichnet, dass das Lösungsmittel, in dessen Medium man die Reaktionen durchführt, ausgewählt wird unter den Ethern, den aromatischen Kohlenwasserstoffen und den Gemischen dieser Lösungsmittel.

8. Verfahren gemäss einem der Ansprüche 1 bis 7, dadurch gekennzeichnet, dass der Protonendonator ausgewählt wird unter 2,4-Dimethyl-6-tert.-butylphenol, Collidinhydrochlorid, Acridinhydrochlorid, Diisopropylaminhydrochlorid, Isopropylethylaminhydrochlorid, Hexamethylentetraminhydrochlorid, Collidinhydrojodid, wasserfreiem Siliciumdioxid, einem Gemisch aus wasserfreiem Siliciumdioxid und wasserfreier Chlorwasserstoffsäure, Ethylacetylacetat, 3,3,6,9,9-Pentamethyl-2,10-diazabicyclo-[4,4,0]-1-decenhydrochlorid und Wasser.

9. Verfahren gemäss einem der Ansprüche 1, 2 und 4 bis 8, dadurch gekennzeichnet, dass der Rest Y ausgewählt wird unter den Dialkyloxyphosphonylresten, den Dialkylboridresten, dem Ethylendioxylboridrest, dem Dimethyl-tert.-butylsilylrest und dem Trimethylsilylrest.

10. Als neue, industrielle Produkte die Produkte der Formel (IV) gemäss Anspruch 1.

**Patentansprüche** für den Vertragsstaat: AT

1. Verfahren zur Epimerisierung von Alkylestern der trans-Chrysanthemumsäure, dadurch gekennzeichnet, dass man bei niedriger Temperatur in wasserfreiem Milieu in dem Medium eines Lösungsmittels einen Alkylester der trans-Chrysanthemumsäure der Formel (II)

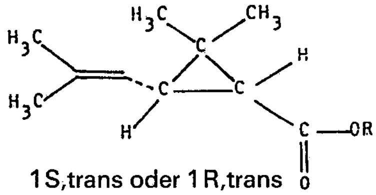

(II)

1 S,trans oder 1 R,trans

worin R einen Alkylester mit 1 bis 6 Kohlenstoffatomen bedeutet, mit einer starken Alkalibase umsetzt, um eine Verbindung der allgemeinen Formel (III)

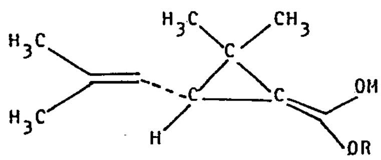

(III)

zu erhalten, worin M ein Alkalimetall bedeutet und R wie vorstehend definiert ist, die Verbindung der allgemeinen Formel (III) der Einwirkung eines Reagens Y-A unterzieht, worin A ein Chlor-, Brom- oder Jodatom oder ein anderes, sich von einer Wasserstoffsäure ableitendes Anion bedeutet und Y einen organischen Rest darstellt, derart, dass das Reagens Y-A befähigt ist, ein Ketalketen zu bilden, um eine Verbindung der Formel (IV)

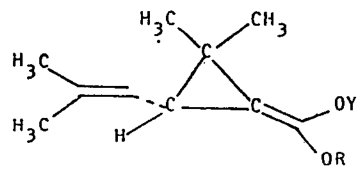

(IV)

zu erhalten, worin Y und R die vorstehend angegebenen Bedeutungen besitzen, die Verbindung (IV) bei niedriger Temperatur in dem Medium eines Lösungsmittels in wasserfreiem Milieu der Einwirkung eines Protonendonators unterzieht, eine Mischung aus dem Alkylester der cis-Chrysanthemumsäure und dem Alkylester der trans-Chrysanthemumsäure erhält und schliesslich den Alkylester der cis-Chrysanthemumsäure (I)

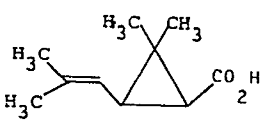

(I)

1 R, cis oder 1 S,cis

aus diesem Gemisch abtrennt und dann diese cis-Säure (I) mit Hilfe eines an sich bekannten Verfahrens isoliert.

2. Verfahren gemäss Anspruch 1 zur Epimerisierung von Alkylestern der 1 S,trans-Chrysanthemumsäure, dadurch gekennzeichnet, dass man von einem Alkylester der 1 S,trans-Chrysanthemumsäure (II) ausgeht und die 1 R,cis-Chrysanthemumsäure (I) erhält.

3. Variante des Verfahrens gemäss Anspruch 1, dadurch gekennzeichnet, dass man direkt die Verbindung (III) bei niedriger Temperatur in dem Medium eines Lösungsmittels in wasserfreiem Milieu der Einwirkung eines Protonendonators unterzieht, um ein Gemisch des Alkylesters der cis-Chrysanthemumsäure und des Alkylesters der trans-Chrysanthemumsäure zu erhalten, danach den Alkylester der cis-Chrysanthemumsäure abtrennt und die cis-Chrysanthemumsäure mit Hilfe eines an sich bekannten Verfahrens, wie in dem Verfahren gemäss Patentanspruch 1, isoliert.

4. Verfahren gemäss einem der Ansprüche 1, 2 oder 3, dadurch gekennzeichnet, dass der Rest R einen Methyl- oder Ethylrest bedeutet.

5. Verfahren gemäss einem der Ansprüche 1 bis 4, dadurch gekennzeichnet, dass die starke Alkalibase Lithiumdiisopropylamid ist.

6. Verfahren gemäss einem der Ansprüche 1 bis 5, dadurch gekennzeichnet, dass die niedrige Temperatur, bei der man die Reaktionen durchführt, zwischen −20 und −70°C liegt.

7. Verfahren gemäss einem der Ansprüche 1 bis 6, dadurch gekennzeichnet, dass das Lösungsmittel, in dessen Medium man die Reaktionen durchführt, ausgewählt wird unter den Ethern, den aromatischen Kohlenwasserstoffen und den Gemischen dieser Lösungsmittel.

8. Verfahren gemäss einem der Ansprüche 1 bis 7, dadurch gekennzeichnet, dass der Protonendonator ausgewählt wird unter 2,4-Dimethyl-6-tert.-butylphenol, Collidinhydrochlorid, Acridinhydrochlorid, Diisopropylaminhydrochlorid, Isopropylethylaminhydrochlorid, Hexamethylentetraminhydrochlorid, Collidinhydrojodid, wasserfreiem Siliciumdioxid, einem Gemisch aus wasserfreiem Siliciumdioxid und wasserfreier Chlorwasserstoffsäure, Ethylacethylacetat, 3,3,6,9,9-Pentamethyl-2,10-diazabicyclo-[4,4,0]-1-decenhydrochlorid und Wasser.

9. Verfahren gemäss einem der Ansprüche 1, 2 und 4 bis 8, dadurch gekennzeichnet, dass der Rest Y ausgewählt wird unter den Dialkoxyphosphonylresten, den Dialkylboridresten, dem Ethylendioxylboridrest, dem Dimethyltert.-butylsilylrest und dem Trimethylsilylrest.

**Claims** for the contracting States: BE, CH, DE, GB, IT, LI, LU, NL, SE

1. Epimerisation process for alkyl esters of trans chrysanthemic acid, characterized in that an alkyl ester of trans chrysanthemic acid with the formula (II)

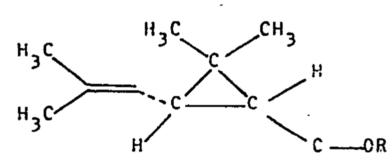

(II)

1 S,trans or 1 R,trans

in which R represents an alkyl radical containing 1 to 6 carbon atoms, is made to react, at low temperature, in an anhydrous medium, in a solvent, with a strong alkaline base, so as to obtain a compound with the general formula (III)

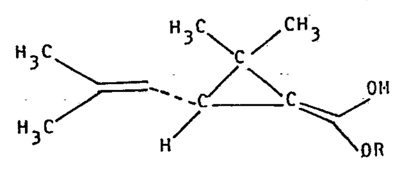

(III)

in which M represents an alkali metal and R is defined as previously, which compound with the general formula (III) is submitted to the action of a reagent Y-A, in which formula A represents a chlorine, bromine or iodine atom or another anion derived from a hydracid and Y represents an organic radical such that the reagent Y-A is able to form a ketene from ketal, so as obtain a compound with the formula (IV)

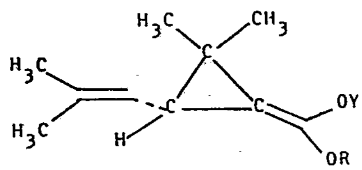

(IV)

in which Y and R retain the significances already given, which compound with the formula (IV) is submitted at low temperature, in a solvent, in an anhydrous medium, to the action of a proton donor, thus obtaining a mixture of alkyl ester of cis chrysanthemic acid and of alkyl ester of trans chrysanthemic acid and finally separating the alkyl ester of cis chrysanthemic acid (I)

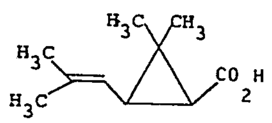

(I)

1R,cis or 1S,cis

from this mixture, then isolating the said cis acid (I) by a process known *per se*.

2. Process according to claim 1 for epimerisation of alkyl esters of 1S,trans chrysanthemic acid, characterized in that one starts with an alkyl ester of 1S,trans chrysanthemic acid (II) and obtains 1R,cis chrysanthemic acid (I).

3. Variant of the process according to claim 1, characterized in that the compound with the formula (III) is submitted directly, at low temperature, in a solvent, in an anhydrous medium, to the action of a proton donor, so as to obtain a mixture of alkyl ester of cis chrysanthemic acid and of alkyl ester of trans chrysanthemic acid, then the alkyl ester of cis chrysanthemic acid is separated and the cis chrysanthemic acid is isolated by a process known *per se*, as in the process in claim 1.

4. Process according to one of claims 1, 2 or 3, characterized in that the radical R is a methyl or ethyl radical.

5. Process according to one of claims 1 to 4, characterized in that the strong alkaline base is lithium diisopropyl amide.

6. Process according to one of claims 1 to 5, characterized in that the low temperature at which the reactions are carried out is between −20 and −70°C.

7. Process according to one of claims 1 to 6, characterized in that the solvent in which the reactions are carried out is chosen from the group constituted by ethers, aromatic hydrocarbons and the mixtures of these solvents.

8. Process according to one of claims 1 to 7, characterized in that the proton donor is chosen from the group constituted by 2,4-dimethyl-6-tertbutylphenol, collidine hydrochloride, acridine hydrochloride, diisopropylamine hydrochloride, isopropyl ethylamine hydrochloride, hexamethylene tetramine hydrochloride, collidine hydriodide, anhydrous silica, a mixture of anhydrous silica and anhydrous hydrochloric acid, ethyl acetyl acetate, the hydrochloride of 3,3,6,9,9-pentamethyl-2,10-diazabicyclo-[4,4,0]-1-decene and water.

9. Process according to one of claims 1, 2 and 4 to 8, characterized in that the radical Y is chosen from the group constituted by the dialkyloxyphosphonyl radicals, dialkyl boride, ethylene dioxyl boride, dimethyltertbutylsilyl and trimethylsilyl.

10. As new industrial products, the products with the formula (IV), as defined in claim 1.

**Claims** for the contracting State: AT

1. Epimerisation process for alkyl esters of trans chrysanthemic acid, characterized in that an alkyl ester of trans chrysanthemic acid with the formula (II)

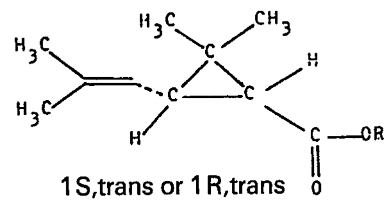

(II)

1S,trans or 1R,trans

in which R represents an alkyl radical containing 1 to 6 carbon atoms, is made to react, at low temperature, in an anhydrous medium, in a solvent, with a strong alkaline base, so as to obtain a compound with the general formula (III)

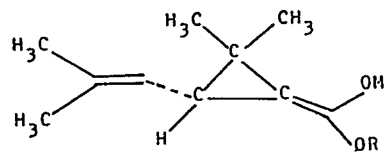

(III)

in which M represents an alkali metal and R is defined as previously, which compound with the general formula (III) is submitted to the action of a reagent Y-A, in which formula A represents a chlorine, bromine or iodine atom or another anion derived from a hydracid and Y represents an organic radical such that the reagent Y-A is able to form a ketene from ketal, so as to obtain a compound with the formula (IV)

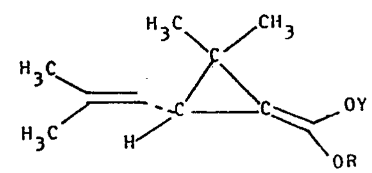

(IV)

in which Y and R retain the significances already given, which compound with the formula (IV) is submitted at low temperature, in a solvent, in an anhydrous medium, to the action of a proton donor, thus obtaining a mixture of alkyl ester of cis chrysanthemic acid and of alkyl ester of trans

chrysanthemic acid and finally separating the alkyl ester of cis chrysanthemic acid (I)

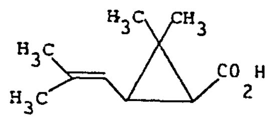

(I)

1R,cis or 1S,cis

from this mixture, then isolating the said cis acid (I) by a process known *per se*.

2. Process according to claim 1 for epimerisation of alkyl esters of 1S,trans chrysanthemic acid, characterized in that one starts with an alkyl ester of 1S,trans chrysanthemic acid (II) and obtains 1R,cis chrysanthemic acid (I).

3. Variant of the process according to claim 1, characterized in that the compound with the formula (III) is submitted directly, at low temperature, in a solvent, in an anhydrous medium, to the action of a proton donor, so as to obtain a mixture of alkyl ester of cis chrysanthemic acid and of alkyl ester of trans chrysanthemic acid, then the alkyl ester of cis chrysanthemic acid is separated and the cis chrysanthemic acid is isolated by a process known *per se*, as in the process in claim 1.

4. Process according to one of claims 1, 2 or 3, characterized in that the radical R is a methyl or ethyl radical.

5. Process according to one of claims 1 to 4, characterized in that the strong alkaline base is lithium diisopropyl amide.

6. Process according to one of claims 1 to 5, characterized in that the low temperature at which the reactions are carried out is between −20 and −70°C.

7. Process according to one of claims 1 to 6, characterized in that the solvent in which the reactions are carried out is chosen from the group constituted by ethers, aromatic hydrocarbons and the mixtures of these solvents.

8. Process according to one of claims 1 to 7, characterized in that the proton donor is chosen from the group constituted by 2,4-dimethyl-6-tertbutylphenol, collidine hydrochloride, acridine hydrochloride, diisopropylamine hydrochloride, isopropyl ethylamine hydrochloride, hexamethylene tetramine hydrochloride, collidine hydriodide, anhydrous silica, a mixture of anhydrous silica and anhydrous hydrochloric acid, ethyl acetyl acetate, the hydrochloride of 3,3,6,9,9-pentamethyl-2,10-diazabicyclo-[4,4,0]-1-decene and water.

9. Process according to one of claims 1, 2 and 4 to 8, characterized in that the radical Y is chosen from the group constituted by the dialkyloxyphosphonyl radicals, dialkyl boride, ethylene dioxyl boride, dimethyltertbutylsilyl and trimethylsilyl.